# EUROPÄISCHE PATENTSCHRIFT

(11) **EP 0 684 810 B1**
(45) Veröffentlichungstag und Bekanntmachung des Hinweises auf die Patenterteilung: **18.06.1997**
(21) Anmeldenummer: 94907559.2
(22) Anmeldetag: 12.02.1994
(51) Int. Cl.: A61K 7/13

(54) **VERWENDUNG VON 1,8-DIHYDROXYNAPHTHALINEN ALS OXIDATIONSFARBSTOFFVORPRODUKTE IN OXIDATIONSFÄRBEMITTELN**
USE OF 1,8-DIHYDROXYNAPHTHALENES AS OXIDATION DYE PRELIMINARY PRODUCTS IN OXIDATION DYEING AGENTS
UTILISATION DE 1,8-DIHYDROXYNAPHTALINES COMME PRODUITS PRELIMINAIRES DE COLORANTS D'OXYDATION DANS DES MATIERES COLORANTES D'OXYDATION

(30) Priorität: 20.02.1993 DE 4305258
(43) Veröffentlichungstag der Anmeldung: 06.12.1995
(73) Patentinhaber: Henkel Kommanditgesellschaft auf Aktien, 40191 Düsseldorf (DE)
(72) Erfinder: KNÜBEL, Georg, D-40229 Düsseldorf (DE); HÖFFKES, Horst, D-40595 Düsseldorf (DE); LIESKE, Edgar, D-40213 Düsseldorf (DE)
(86) Internationale Anmeldenummer: EP9400406
(87) Internationale Veröffentlichungsnummer: WO9418937

(56) Entgegenhaltungen:
- DE-A- 2 160 317
- GB-A- 2 205 111
- US-A- 3 415 608
- US-A- 4 172 703
- CHEMICAL ABSTRACTS, vol. 99, no. 23, 1983, Columbus, Ohio, US; abstract no. 191290w, & TRANS. BR. MYCOL. SOC. Bd. 81, Nr. 1 , 1983 , USA Seiten 29 - 36 M. H. WHEELER 'comparisons of fungal melanin biosynthesis in ascomycetous, imperfect and basidiomycetous fungi'
- CHEMICAL ABSTRACTS, vol. 99, no. 23, 1983, Columbus, Ohio, US; abstract no. 191290w, & Trans. Br. Mycol. Soc. Bd. 81, Nr. 1, 1983, USA, M.H. Wheeler"Comparisons of fungal melanin biosynthesis in ascomycetouy, imperfect and basidiomycetous fungi"

## Beschreibung

Die Erfindung betrifft die Verwendung von 1,8-Dihydroxynaphthalinen als Oxidationsfarbstoffvorprodukte in Oxidationsfärbemitteln zum Färben von Keratinfasern, insbesondere menschlichen Haaren. Die 1,8-Dihydroxynaphthaline eignen sich besonders als Kupplerkomponenten gemeinsam mit Entwicklerkomponenten.

Oxidationsfärbemittel enthalten üblicherweise Oxidationsfarbstoffvorprodukte in einem wäßrigen Träger; als Oxidationsfarbstoffvorprodukte werden Entwicklerkomponenten und Kupplerkomponenten eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Von den zehn möglichen Dihydroxynaphthalin-Isomeren sind bisher neun als Farbstoffvorprodukte in Oxidationsfärbemitteln beschrieben worden. In der folgenden Übersicht werden repräsentative Patentliteraturzitate aufgeführt:
- 1,2-Dihydroxynaphthalin:: JP 83/45401 (Auslegeschrift)
- 1,3-Dihydroxynaphthalin:: GB-A- 2 205 111
- 1,4-Dihydroxynaphthalin:: DE 605 684
- 1,5-Dihydroxynaphthalin:: DE 51 073
- 1,6-Dihydroxynaphthalin:: DE 624 617
- 1,7-Dihydroxynaphthalin:: US 4 172 703
- 1,8-Dihydroxynaphthalin:: ----------
- 2,3-Dihydroxynaphthalin:: US 3 415 608
- 2,6-Dihydroxynaphthalin:: DE 624 617
- 2,7-Dihydroxynaphthalin:: DE 367 690

Die Verwendung von 1,2,5,8-Tetrahydroxynaphthalin-Oxidationsfarbstoffvorprodukt ist aus DE 285 769 bekannt. Die Verwendung von 1,8-Dihydroxynaphthalin in Oxidationsfärbemitteln ist bisher noch nicht beschrieben worden.

In früheren Druckschriften wird angegeben, daß 1,8-Dihydroxynaphthalin unbeständig ist (z.B. Elsevier's Encyclopedia of Organic Chemistry, Elsevier Publishing Company, 1950, Series III, Vol. 12 b, S. 1991).

Überraschenderweise wurde nun gefunden, daß sich 1,8-Dihydroxynaphthalin und bestimmte halogensubstituierte Derivate hervorragend als Oxidationsfarbstoffvorprodukte zur Erzielung von Oxidationsfärbungen eignen und in Oxidationshaarfärbemittel-Zubereitungen ausreichend beständig sind. In ihren färbetechnischen Eigenschaften sind diese 1,8-Dihydroxynaphthaline den o.g. isomeren Dihydroxynaphthalinen dabei deutlich überlegen.

Gegenstand der Erfindung ist die Verwendung von 1,8-Dihydroxynaphthalinen der Formel I 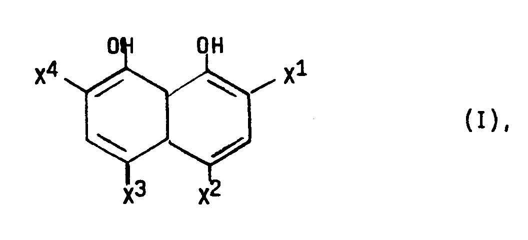 wobei X¹ bis X⁴ unabhängig voneinander für Wasserstoff-, Chlor- oder Bromatome stehen, in Oxidationsfärbemitteln zum Färben von Keratinfasern. Unter Keratinfasern sind Haare, Pelze, Wolle oder Federn zu verstehen.
Insbesondere eignet sich der Grundkörper 1,8-Dihydroxynaphthalin selbst, also diejenige Verbindung, in der X¹ bis X⁴ Wasserstoffe sind.
Ganz besonders eignen sich die 1,8-Dihydroxynaphthaline der Formel I zum Färben von Keratinfasern, wenn sie gemeinsam mit üblichen Entwicklerkomponenten eingesetzt werden.

Ein weiterer Patentgegenstand ist deshalb dier Verwendung von 1,8-Dihydroxynaphtalinen der Fomel I als Kupplerkomponenten in Oxidationsfärbemitteln gemeinsam mit üblichen Entwicklerkomponenten zum Färben von Keratinfasern.

Übliche Entwicklerkomponenten sind z.B. primäre aromatische Amine mit einer weiteren in para- oder ortho-Position befindlichen freien oder substituierten Hydroxy- oder Aminogruppe, Diaminopyridin-Derivate, heterocyclische Hydrazone, 4-Aminopyrazolon-Derivate, 2,4,-5,6-Tetraaminopyrimidin, 2,4,5-Triamino-6-hydroxy-pyrimidin, 5,6-Diamino-2,4-dihydroxypyrimidin und deren Derivate. Einige speziell geeignete Entwicklerkomponenten sind im Beispielteil aufgeführt.

Die 1,8-Dihydroxynaphthaline der Formel I liefern mit den üblichen Entwicklerkomponenten ein breites Spektrum anwendungstechnisch interessanter Oxidationsfarben im Bereich roter, violetter, blauer, schwarzer und grüner Nuancen, die sich wegen ihrer Brillanz und guten Echtheitseigenschaften insbesondere zum Färben von menschlichen Haaren eignen.

Ein weiterer Patentgegenstand sind deshalb Oxidationsfärbemittel zum Färben von menschlichen Haaren enthaltend Oxidationsfarbstoffvorprodukte in einem wasserhaltigen Träger, wobei die 1,8-Dihydroxynaphthaline der Formel I als Kuppler in einer Menge von 0,05 bis 20 mMol sowie übliche Entwicklerkomponenten in einer Menge von 0,05 bis 20 mMol und gegebenenfalls übliche direktziehende Farbstoffe in einer Menge von 0,05 bis 20 mMol, jeweils pro 100 g des Färbemittels enthalten sind.

Die erfindungsgemäßen Haarfärbemittel können neben den 1,8-Dihydroxynaphthalinen der Formel I auch andere bekannte Kupplerkomponenten enthalten, die zur Modifizierung der Farbnuancen und zur Erzeugung natürlicher Farbtöne erforderlich sind. Solche üblichen Kupplerverbindungen sind z.B. meta-Phenylendiaminderivate, Naphthole, Resorcinderivate und Pyrazolone. Gegebenenfalls können auch mehrere bekannte Entwicklerkomponenten und direktziehende Farbstoffe zur weiteren Modifizierung der Farbnuancen eingesetzt werden. Direktziehende Farbstoffe sind z.B. Nitrophenylendiamine, Nitroaminophenole, Anthrachinonfarbstoffe oder Indophenole.
Es ist auch nicht erforderlich, daß die 1,8-Dihydroxynaphthaline der Formel I einheitliche Verbindungen sind. Vielmehr können auch Gemische verschiedener Verbindungen der Formel I verwendet werden.

In den erfindungsgemäßen Haarfärbemitteln werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können. Zur Herstellung der erfindungsgemäßen Färbemittel werden die Oxidationsfarbstoffvorprodukte in einem geeigneten wasserhaltigen Träger eingearbeitet. Solche Träger sind z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Der wasserhaltige Träger enthält üblicherweise Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z. B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Die Bestandteile des wasserhaltigen Trägers werden zur Herstellung der erfindungsgemäßen Haarfärbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht ist. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo verwendet wurde.

Die nachfolgenden Beispiele sollen den Erfindungsgegenstand näher erläutern, ohne ihn jedoch hierauf zu beschränken.

### Beispiele

### Beispiel I

Es wurde ein erfindungsgemäßes Färbemittel in Form einer Haarfärbecreme-Emulsion der folgenden Zusammensetzung hergestellt

| | |
|---|---|
| Fettalkohol C_{12/18} | 10,0 g |
| Fettalkohol C_{12/14} + 2 EO-sulfat (Na-salz, 28 %ig) | 25,0 g |
| Wasser | 60,0 g |
| 1,8-Dihydroxynaphthalin | 6,5 mMol |
| Ammoniumsulfat | 1,0 g |
| konz. Ammoniaklösung | bis pH = 9,5 |
| Wasser | ad 100 g |

Die Bestandteile wurden der Reihe nach miteinander vermischt. Nach Zugabe der Oxidationsfarbstoffvorprodukte und des Inhibitors wurde zunächst mit konzentrierter Ammoniak lösung der pH-Wert der Emulsion auf 9,5 eingestellt, dann wurde mit Wasser auf 100 g aufgefüllt.

Die oxidative Entwicklung der Färbung wurde entweder durch Luftoxidation oder mit Hilfe 3 %iger Wasserstoffperoxidlösung als Oxidationsmittel durchgeführt. Hierzu wurden 100 g der Emulsion mit 50 g Wasserstoffperoxidlösung versetzt und vermischt. Die Färbecreme wurde auf ca. 5 cm lange Strähnen standardisierten, zu 90 % ergrauten, aber nicht besonders vorbehandelten Menschenhaars aufgetragen und dort 30 Minuten, im Falle der Luftoxidation 60 Minuten, bei 27 °C belassen. Nach Beendigung des Färbeprozesses wurde das Haar gespült, mit einem üblichen Haarwaschmittel ausgewaschen und anschließend getrocknet.

Die Luftoxidation ergab eine Nuance im Braunbereich; bei der H₂O₂-Oxidation resultierte eine Nuance im Braunbereich, die rötlich changierte.

### Beispiel II

Es wurden erfindungsgemäße Färbemittel in Form einer Haarfärbecreme-Emulsion der folgenden Zusammensetzung hergestellt:

| | |
|---|---|
| Fettalkohol C_{12/18} | 10,0 g |
| Fettalkohol C_{12/14} + 2 EO-Sulfat, Na-Salz, 28 %ig | 25,0 g |
| Wasser | 60,0 g |
| Komponente E (Entwicklerkomponente) | 6,5 mMol |
| 1,8-Dihydroxynaphthalin (Kupplerkomponente) | 6,5 mMol |
| Natriumsulfit (Inhibitor) | 1,0 g |
| Ammoniumsulfat | 1,0 g |
| konzentrierte Ammoniaklösung | bis pH = 9,5 |
| Wasser | ad 100 g |

Als Komponente E (Entwicklerkomponente) wurden folgende Verbindungen eingesetzt:
- E1:: 3-Methyl-p-aminophenol
- E2:: p-Aminophenol-Hydrochlorid
- E3:: 2,5-Diaminotoluol-Sulfat
- E4:: 2-(2,5-Diaminophenyl)ethanol
- E5:: 1,10-Bis-(2,5-Diaminophenyl)-1,4,7,10-tetraoxadecan Hydrochlorid
- E6:: 2,4,5,6-Tetraaminopyrimidin-Sulfat
- E7:: 2,4,5-Triamino-6-hydroxypyrimidin-Sulfat
- E8:: 5,6-Diamino-2,4-dihydroxypyrimidin-Sulfat

Die Bestandteile wurden der Reihe nach miteinander vermischt. Nach Zugabe der Oxidationsfarbstoffvorprodukte und des Inhibitors wurde zunächst mit konzentrierter Ammoniak lösung der pH-Wert der Emulsion auf 9,5 eingestellt, dann wurde mit Wasser auf 100 g aufgefüllt. Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Die Ergebnisse der Haarfärbeversuche sind der folgenden Tabelle zu entnehmen:

**Tabelle**

| Haarfärbe-Beispiel | Entwicklerkomponente | erhaltene Farbnuancen |
|---|---|---|
| 1 | E 1 | rubin |
| 2 | E 2 | braunviolett |
| 3 | E 3 | blauschwarz |
| 4 | E 4 | blauschwarz |
| 5 | E 5 | schwarzblau |
| 6 | E 6 | schwarzgrün |
| 7 | E 7 | mattblau |
| 8 | E 8 | grün |

Die Haarfärbungen verfügten über hervorragende Farbintensitäten und Echtheitseigenschaften.
Mit der Entwicklerkomponente E 3 (2,5-Diaminotoluolsulfat) wurden Vergleichsversuche gegenüber anderen Dihydroxynaphthalin-Isomeren durchgeführt. Es zeigte sich, daß die mit der Kombination E 3 / 1,8-Dihydroxynaphthalin erzielten Ausfärbungen den mit den Kombinationen E 3 / 1,5-, 1,6- oder 1,7-Dihydroxynaphthalin erzielten Ausfärbungen hinsichtlich Farbtiefe und Lichtechtheit signifikant überlegen sind.

## Patentansprüche

1. Verwendung von 1,8-Dihydroxynaphthalinen der Formel I 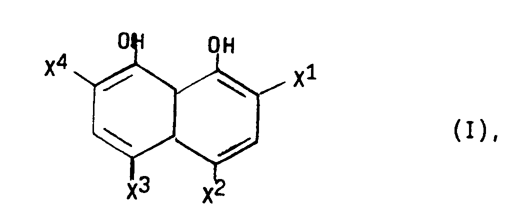 wobei X¹ bis X⁴ unabhängig voneinander für Wasserstoff-, Chlor- oder Bromatome stehen, in Oxidationsfärbemitteln zum Färben von Keratinfasern.

2. Verwendung nach Anspruch 1, dadurch gekennzeichnet, daß die Verbindung der Formel I 1,8-Dihydroxynaphthalin ist.

3. Verwendung von 1,8-Dihydroxynaphthalinen der Formel I gemäß Anspruch 1 als Kupplerkomponenten in Oxidationsfärbemitteln gemeinsam mit üblichen Entwicklerkomponenten.

4. Oxidationsfärbemittel zum Färben von menschlichen Haaren, enthaltend Oxidationsfarbstoffvorprodukte in einem wasserhaltigen Träger, dadurch gekennzeichnet, daß die 1,8-Dihydroxynaphthaline der Formel I als Kuppler in einer Menge von 0,05 bis 20 mMol sowie übliche Entwicklerkomponenten in einer Menge von 0,05 bis 20 mMol und gegebenenfalls übliche direktziehende Farbstoffe in einer Menge von 0,05 bis 20 mMol pro 100 g des Färbemittels enthalten sind.

## Claims

1. The use of 1,8-dihydroxynaphthalenes corresponding to formula I: 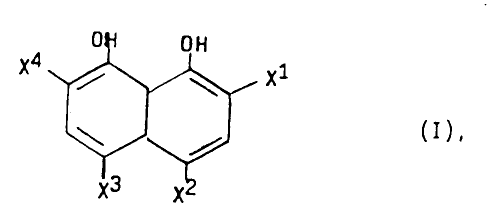 in which X¹ to X⁴ independently of one another represent hydrogen, chlorine or bromine atoms,
in oxidation colorants for colouring keratin fibres.

2. The use claimed in claim 1, characterized in that the compound corresponding to formula I is 1,8-dihydroxynaphthalene.

3. The use of 1,8-dihydroxynaphthalenes corresponding to formula I in claim 1 as secondary intermediates in oxidation colorants in conjunction with typical primary intermediates.

4. Oxidation colorants for colouring human hair containing oxidation dye precursors in a water-containing carrier, characterized in that the 1,8-dihydroxynaphthalenes corresponding to formula I are present as secondary intermediates in a quantity of 0.05 to 20 mmoles together with typical primary intermediates in a quantity of 0.05 to 20 mmoles and, optionally, typical substantive dyes in a quantity of 0.05 to 20 mmoles per 100 g of the colorant.

## Revendications

1. Utilisation de 1,8-dihydroxynaphtalènes de la formule I 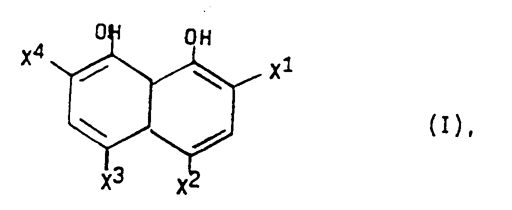 dans laquelle X¹ à X⁴ représentent indépendamment les uns des autres un atome d'hydrogène, de chlore ou de brome, dans des teintures d'oxydation pour la coloration de fibres kératiniques.

2. Utilisation selon la revendication 1, caractérisée en ce que le composé de la formule I est le 1,8-dihydroxynaphtalène.

3. Utilisation de 1,8-dihydroxynaphtalènes de la formule I conformément à la revendication 1, comme composants copulateurs dans des teintures d'oxydation, conjointement avec des composants développateurs usuels.

4. Teintures d'oxydation pour la coloration des cheveux humains, contenant des précurseurs de colorants d'oxydation dans un vecteur aqueux, caractérisées en ce que les 1,8-dihydroxynaphtalènes de la formule I sont contenus comme copulateurs dans une proportion de 0,05 à 20 mmoles conjointement avec des composants développateurs usuels dans une proportion de 0,05 à 20 mmoles et, éventuellement, avec des colorants montant directement sur la fibre usuels dans une proportion de 0,05 à 20 mmoles pour 100 g de la teinture.
